# EUROPEAN PATENT APPLICATION

(11) **EP 2 299 273 A1**
(43) Date of publication of application: **23.03.2011**
(21) Application number: 09758399.1
(22) Date of filing: 04.06.2009
(51) Int. Cl.: G01N 33/579, G01N 21/49, G01N 21/82, G01N 33/48

(54) **METHOD OF ASSAYING PHYSIOLOGICALLY ACTIVE SUBSTANCE OF BIOLOGICAL ORIGIN, KIT FOR ASSAYING PHYSIOLOGICALLY ACTIVE SUBSTANCE OF BIOLOGICAL ORIGIN AND APPARATUS FOR ASSAYING PHYSIOLOGICALLY ACTIVE SUBSTANCE OF BIOLOGICAL ORIGIN**

(30) Priority: 05.06.2008 JP 2008148600
(71) Applicant: Kowa Company, Ltd., Nagoya-shi, Aichi 460-8625 (JP)
(72) Inventor: YABUSAKI, Katsumi, Shizuoka 431-2103 (JP)
(74) Representative: Elsy, David
(86) International application number: PCT/JP2009/060282
(87) International publication number: WO 2009/148132

(57) **Abstract**

Provided is a technique whereby a physiologically active substance of biological origin such as an endotoxin or β-D-glucan can be more conveniently and accurately detected and the concentration thereof can be determined even in the case of using a sample which contains a substance affecting LAL activity. For example, a sample is brought into contact with a substance (2) capable of adsorbing an endotoxin and thus the endotoxin contained in the sample is preliminarily adsorbed. Next, the sample per se is washed away and the adsorbed endotoxin (4) is reacted with LAL. Then the gelation of LAL or the formation of gel particles (5) is detected to thereby detecting the endotoxin and determining the concentration thereof.

## Description

### Technical Field

The present invention relates to a method of determining a physiologically active substance of biological origin, a kit for determining a physiologically active substance of biological origin, and an apparatus for determining a physiologically active substance of biological origin, each of which is used for detecting a physiologically active substance of biological origin in an analyte sample containing a substance which affects an LAL activity or for determining the concentration of the substance.

### Background Art

Endotoxin is a lipopolysaccharide present in a cell wall of a Gram-negative bacterium and is the most typical pyrogen. If a transfusion, a medicine for injection, or blood contaminated with endotoxin is introduced into the human body, endotoxin may induce severe side effects such as fever and shock. Therefore, it has been required that the above-mentioned medicine or the like is kept not to be contaminated with endotoxin.

By the way, a limulus amoebocyte lysate (hereinafter, also referred to as "LAL") contains a serine protease which is activated by endotoxin. In the case where LAL reacts with endotoxin, a coagulogen present in LAL is hydrolyzed into a coagulin by an enzyme cascade of the serine protease activated depending on the amount of endotoxin, and the coagulin is associated with one another to form an insoluble gel. By using the characteristic of LAL, endotoxin can be detected with a high sensitivity.

Meanwhile, β-D-glucan is a polysaccharide which constitutes a cell membrane characteristic of a fungus. Determination of β-D-glucan is effective, for example, for screening of infectious diseases due to a variety of fungi including not only fungi which are frequently observed in general clinical practices, such as Candida, Spergillus, and Cryptococcus, but also rare fungi.

In determination of β-D-glucan, by using the characteristic of a limulus amoebocyte lysate to coagulate (coagulate to form a gel) by β-D-glucan, β-D-glucan can be detected with a high sensitivity.

A method for detection or concentration determination of a physiologically active substance of biological origin (hereinafter, also referred to as a predetermined physiologically active substance) which can be detected by a limulus amoebocyte lysate, such as endotoxin or β-D-glucan includes a semi-quantitative gelation method involving leaving a mixture obtained by mixing a sample to be used for detection or concentration determination of the predetermined physiologically active substance (hereinafter, also simply referred to as "determination of the predetermined physiologically active substance") (hereinafter, also referred to as "sample for determination of the predetermined physiologically active substance") with LAL to stand still; inverting the container after a lapse of a predetermined time; and judging whether the sample gets gelation or not based on the presence or absence of dipping of the sample to examine whether or not the sample contains the predetermined physiologically active substance above a certain concentration. There are also given a turbidimetric method involving analyzing a sample with time by determining the turbidity of the sample caused by gel formation by a reaction between LAL and the predetermined physiologically active substance, a colorimetric method using a synthetic substrate which is hydrolyzed by an enzyme cascade to develop a color, and the like.

In the case where the predetermined physiologically active substance is determined by the above-mentioned turbidimetric method, a mixture of a determination sample containing the predetermined physiologically active substance and LAL is produced in a dry-heat-sterilized glass determination cell. Then, gelation of the mixture is optically determined from the outside. However, the turbidimetric method may require a very long time for gelation of LAL particularly in a sample containing the predetermined physiologically active substance at a low concentration. To solve the problem, a method which can measure the predetermined physiologically active substance in a short time has been required. There has been proposed a laser light scattering particle counting method or a stirring turbidimetric method capable of forming gel fine particles by stirring a mixture of a determination sample of the predetermined physiologically active substance and LAL using a magnetic stirring bar, for example, and determining the presence of the predetermined physiologically active substance in the sample in a short time based on the intensity of laser light scattered by the gel fine particles or based on the intensity of light transmitted through the mixture.

By the way, LAL gelates with the predetermined physiologically active substance by the activity of serine protease, and hence the presence of a substance which affects an LAL activity greatly affects quantification of the predetermined physiologically active substance. For example, in the case where the sample is blood, blood itself includes coagulation and fibrinolytic systems, which are mainly formed of a serine protease cascade very similar to a clotting enzyme cascade of LAL, and the systems are known to interact with serine protease in LAL.

Based on the above-mentioned facts, the following pretreatment may be carried out to suppress the activity of serine protease in blood. For example, in order to remove erythrocytes which inhibit optical determination, a blood analyte is lightly centrifuged to prepare plasma containing a large amount of platelets (platelet-rich plasma: PRP). Thereafter, the PRP is collected, and an appropriate surfactant is added, followed by heating to deactivate enzymes in the sample.

However, the pretreatment includes cumbersome steps, resulting in variations in data among different testers, for example. Moreover, a previous report shows that the predetermined physiologically active substance is bound to or included in blood components other than PRP (such as leukocytes, erythrocytes, and platelets which are not collected as PRP) and determination for whole blood is more desirable.

Also in the case where the sample is not blood, a protease, a protease inhibitor, a protein denaturant, a surfactant, or a chelating agent changes the action of LAL. Therefore, in the case where an obj ect to be determined contains such substance, the accuracy of determination may lower. Many of medicines for injection are used for treatment of diseases in blood coagulation and fibrinolytic systems, and it may be difficult to accurately measure the concentration of the predetermined physiologically active substance in such analytes.

Meanwhile, to solve the above-mentioned disadvantages, there has been proposed a PyroSep method involving immobilizing PyroSep for adsorbing the predetermined physiologically active substance on the surface of a carrier such as cellulose beads, reacting a sample containing the predetermined physiologically active substance with the carrier to adsorb the predetermined physiologically active substance, washing the carrier with a liquid free of the predetermined physiologically active substance, suspending the carrier in a buffer, and determining the predetermined physiologically active substance by the turbidimetric method.

However, as described above, this method requires a step of taking out the carrier from the column to react the predetermined physiologically active substance adsorbed to PyroSep with LAL, and it is difficult to facilitate determination of the predetermined physiologically active substance.

### Citation List

### Patent Literature

PTL 1: JP 2004-061314 A
PTL 2: JP 10-293129 A
PTL 3: JP 05-287496 A
PTL 4: JP 04-59578 B

### Summary of Invention

### Technical Problem

The present invention has been developed in view of the above-mentioned problem. An object of the present invention is to provide a technology for achieving more convenient and accurate detection and concentration determination of a predetermined physiologically active substance even in the case where a sample contains a substance affecting an LAL activity.

### Solution to Problem

The inventors of the present invention have made intensive studies to solve the above-mentioned problem, and as a result, found that: 1) a predetermined physiologically active substance can be concentrated in an observation region by binding a substance which adsorbs the predetermined physiologically active substance only to the observation region and passing the predetermined physiologically active substance through the observation region; 2) when LAL is added to the region to react with the predetermined physiologically active substance, the predetermined physiologically active substance adsorbed and concentrated in the region causes gelation of LAL; 3) the predetermined physiologically active substance can be quantified by determining the turbidity of the sample; 4) even if the sample contains a substance which affects an LAL activity, the effect of the substance can be avoided by washing the determination system with water after concentration of the predetermined physiologically active substance in the observation region; and 5) the determination may be carried out using one-tenth the amount of LAL in some cases in the turbidimetric method because the observation region can be easily minified by improving the determination method, and completed the present invention by combining the findings.

The greatest feature of the present invention is to measure the predetermined physiologically active substance by preliminarily adsorbing the predetermined physiologically active substance in the sample by bringing the sample into contact with a substance capable of adsorbing the predetermined physiologically active substance, removing the sample itself by washing, and detecting gelation or formation of gel particles of LAL caused by a reaction between the adsorbed predetermined physiologically active substance and LAL.

To be more specific, the present invention is a method of determining a physiologically active substance of biological origin, which is used for detecting the physiologically active substance of biological origin in a sample or determining the concentration of the physiologically active substance of biological origin in the sample by reacting the physiologically active substance of biological origin in the sample with LAL which is a limulus amoebocyte lysate, including: an adsorption step of adsorbing the physiologically active substance of biological origin in the sample to a predetermined adsorptive substance capable of adsorbing the physiologically active substance of biological origin; a removal step of removing the sample excluding the physiologically active substance of biological origin adsorbed to the adsorptive substance in the adsorption step from the adsorptive substance, which is carried out after the adsorption step; a reaction step of reacting the physiologically active substance of biological origin adsorbed to the adsorptive substance in the adsorption step with LAL, which is carried out after the removal step; and a detection step of optically detecting gelation or formation of gel particles of LAL in the reaction step.

According to this method, it is possible to adsorb only the predetermined physiologically active substance in the sample to the adsorptive substance depending on the concentration of the substance in the adsorption step. Even if the sample contains a substance which affects an LAL activity, the effect can be easily eliminated because, in the removal step, the sample containing the predetermined physiologically active substance not adsorbed to the adsorptive substance is removed from the adsorptive substance.

In other words, in the adsorption step and the removal step, the predetermined physiologically active substance in the sample can be concentrated. Moreover, in the reaction step, only the predetermined physiologically active substance can be reacted with LAL depending on the concentration because the predetermined physiologically active substance adsorbed to the adsorptive substance is reacted with LAL, and the concentration of the predetermined physiologically active substance can be determined more easily and accurately in the detection step.

Moreover, the amount of LAL used can be reduced because the predetermined physiologically active substance concentrated in the above-mentioned step is reacted with LAL.

Further, in the present invention, the physiologically active substance of biological originmaybe endotoxin or β-D-glucan.

In such case, detection or concentration determination of endotoxin which is the most typical pyrogen can be carried out more accurately, and it is possible to suppress entryof a transfusion, a medicine for injection, or blood contaminated with endotoxin into the human body to induce side effects. Similarly, detection or concentration determination of β-D-glucan can be carried out more accurately, and it is possible to carry out more accurate screening of infectious diseases due to a variety of fungi including not only fungi which are frequently observed in general clinical practices, such as Candida, Spergillus, and Cryptococcus, but also rare fungi.

It should be noted that, in the present invention, examples of the predetermined adsorptive substance include calcium carbonate, aluminum oxide, and hydroxyapatite. When such inorganic substance is selected as the predetermined adsorptive substance, good adsorbability can be achieved regardless of the type of the physiologically active substance of biological origin.

It should be noted that, in the present invention, examples of the predetermined adsorptive substance suitable in the case where the physiologically active substance of biological origin includes endotoxin include polymyxin B, polylysine, polyornithine, polyethyleneimine, a kind of silane coupling agent containing an amino group, histidine, and an anti-endotoxin antibody.

Further, in the present invention, examples of the predetermined adsorptive substance suitable in the case where the physiologically active substance of biological origin includes β-D-glucan include an alkyl group-containing hydrophobic substance, a derivative of the alkyl group-containing hydrophobic substance, aniline blue, cellulose, a derivative of cellulose, glucan, and a derivative of glucan.

Further, the present invention may be a kit for determining a physiologically active substance of biological origin, which is used for optical detection of gelation or formation of gel particles caused by a reaction between the physiologically active substance of biological origin in a sample and LAL which is a limulus amoebocyte lysate, in which a predetermined adsorptive substance capable of adsorbing the physiologically active substance of biological origin is bound to the inside of a container or tubular member formed of a material capable of transmitting light to be used for the optical detection can be transmitted.

Accordingly, when a sample containing the predetermined physiologically active substance is introduced into the kit for determining a predetermined physiologically active substance according to the present invention, the predetermined physiologically active substance can be adsorbed to the adsorptive substance in the inside of a container or a circular member. Moreover, the sample can be removed from the adsorptive substance simply by discharging the sample from the inside of the container or tubular member and washing the container or tubular member. In addition, when an LAL reagent is introduced into the kit for determining a predetermined physiologically active substance according to the present invention, it is possible to react the adsorbed and concentrated predetermined physiologically active substance with LAL to promote gelation or formation of gel particles of LAL.

In addition, the container or tubular member according to the present invention can transmit light to be used for optical detection, and hence gelation or formation of gel particles in the container or tubular member can be detected by making light enter from the outside without further treatment.

The volume of the container or tubular member according to the present invention may be changed very freely. Therefore, the amount of LAL required for determination of the predetermined physiologically active substance can be reduced by appropriately adjusting the volume and binding range of the adsorptive substance.

Further, in the container according to the present invention, the inside of the containermaybe equipped with a stainless steel magnetic stirring bar, on which an oxide film with a thickness of 100 nm or more is formed. By doing so, the magnetic stirring bar can be rotated by applying an electromagnetic force to the magnetic stirring bar from the outside to uniformize the reaction between the predetermined physiologically active substance adsorbed to the adsorptive substance and LAL and to promote the reaction. Further, the magnetic stirring bar used for the present invention has an oxide film with a sufficient thickness on its surface, and hence it is possible to avoid elution of an iron ion in the mixture of the predetermined physiologically active substance adsorbed to the adsorptive substance and LAL to inhibit the activity of the predetermined physiologically active substance.

It should be noted that the above-mentioned oxide film is particularly required for the case where the predetermined physiologically active substance includes endotoxin and a sample free of a buffer, a protein, or an amino acid which adsorbs iron is determined. In the case where the predetermined physiologically active substance includes β-D-glucan or in the case where a blood sample is determined, the oxide film is not particularly required. Meanwhile, the thickness of the oxide film is preferably 1 µm or less. This is because, if the thickness of the oxide film exceeds 1 µm, the oxide film is easily detached (peeled) from the surface of the instrument, and elution of an iron ion due to the detachment of the oxide film may affect determination.

Further, the present invention may be an apparatus for determining a physiologically active substance of biological origin, which is used for detecting the physiologically active substance of biological origin in a sample or determining the concentration of the physiologically active substance of biological origin in the sample by optical detection of gelation or formation of gel particles caused by a reaction between the physiologically active substance of biological origin in the sample and LAL which is a limulus amoebocyte lysate, including: a reaction container which is formed of a material where light to be used for the optical detection can be transmitted and has an adsorption part for adsorbing the physiologically active substance of biological origin in the sample in the inside of the container; adsorption means for adsorbing the physiologically active substance of biological origin in the sample to the adsorption part by introducing the sample into the reaction container; removal means for removing the sample excluding the physiologically active substance of biological origin adsorbed to the adsorption part from the reaction container; LAL introduction means for introducing LAL into the reaction container in which the physiologically active substance of biological origin in the sample is adsorbed to the adsorptionpart; and detection means for optically detecting gelation or formation of gel particles of LAL introduced by the LAL introduction means.

According to the apparatus, it is possible to adsorb only the predetermined physiologically active substance in the sample to the adsorption part in the reaction container depending on the concentration of the substance. Even if the sample contains a substance which affects an LAL activity, the effectcanbe eliminated because the sample containing the predetermined physiologically active substance not adsorbed to the adsorptive substance is removed from the reaction container by the removal means. In other words, in the present invention, the predetermined physiologically active substance in the sample canbe concentrated in the reaction container. Moreover, LAL is introduced into the reaction container by the LAL introduction means to react the predeterminedphysiologically active substance adsorbed to the adsorption part with LAL, and hence it is possible to react only the predetermined physiologically active substance with LAL depending on the concentration and to measure the concentration of the predetermined physiologically active substance more accurately by the detection means.

The amount of LAL used can be reduced because the predetermined physiologically active substance concentrated in the above step is reacted with LAL. The volume of the reaction container may be changed very freely, and the amount of LAL required for determination of the predetermined physiologically active substance can be further reduced by appropriately adjusting the volume and the position range of the adsorption part.

It should be noted that the apparatus for determining a physiologically active substance of biological origin of the present invention may further include: the stirring means which includes a stainless steel magnetic stirring bar, on which an oxide film with a thickness of 100 nm or more is formed, in the reaction container, and stirring the sample by rotating the magnetic stirring bar by applying an electromagnetic force to the magnetic stirring bar from the outside of the reaction container. It should be noted that the above-mentioned oxide film is particularly required for the case where the predetermined physiologically active substance includes endotoxin and a sample free of a buffer, a protein, or an amino acid which adsorbs iron is determined. In the case where the predetermined physiologically active substance includes β-D-glucan or in the case where a blood sample is determined, the oxide film is not particularly required. Meanwhile, the thickness of the oxide film is preferably 1 µm or less. This is because, if the thickness of the oxide film exceeds 1 µm, the oxide film is easily detached (peeled) from the surface of the instrument, and elution of an iron ion due to the detachment of the oxide film may affect determination.

Further, the adsorption part may be formed by binding at least one of calcium carbonate, aluminum oxide, and hydroxyapatite to the reaction container.

Further, when the physiologically active substance of biological origin includes endotoxin, the adsorption part may be formed by binding at least one of polymyxin B, polylysine, polyornithine, polyethyleneimine, a kind of silane coupling agent containing an amino group, histidine, and an anti-endotoxin antibody to the reaction container.

Further, when the physiologically active substance of biological origin includes β-D-glucan, the adsorption part may be formed by binding at least one of an alkyl group-containing hydrophobic substance, a derivative of the alkyl group-containing hydrophobic substance, aniline blue, cellulose, a derivative of cellulose, glucan, and a derivative of glucan to the reaction container.

It should be noted that the above-mentioned means for solving the problems of the present invention may be combined to a maximum extent.

### Advantageous Effects of Invention

In the present invention, it is possible to detect a physiologically active substance of biological origin such as endotoxin or β-D-glucan in a sample accurately or to measure the concentration of the physiologically active substance of biological origin accurately by concentrating the physiologically active substance of biological origin without cumbersome processes even if the sample contains a substance which affects an LAL activity.

### Brief Description of Drawings

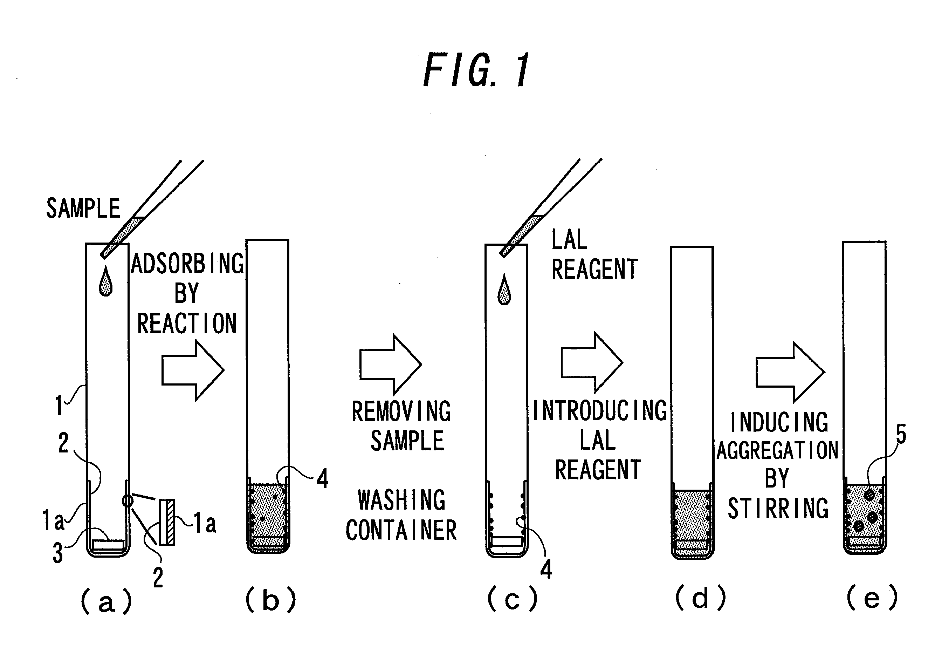

[FIGS. 1] Diagrams each illustrating determination of endotoxin in Examples of the present invention.

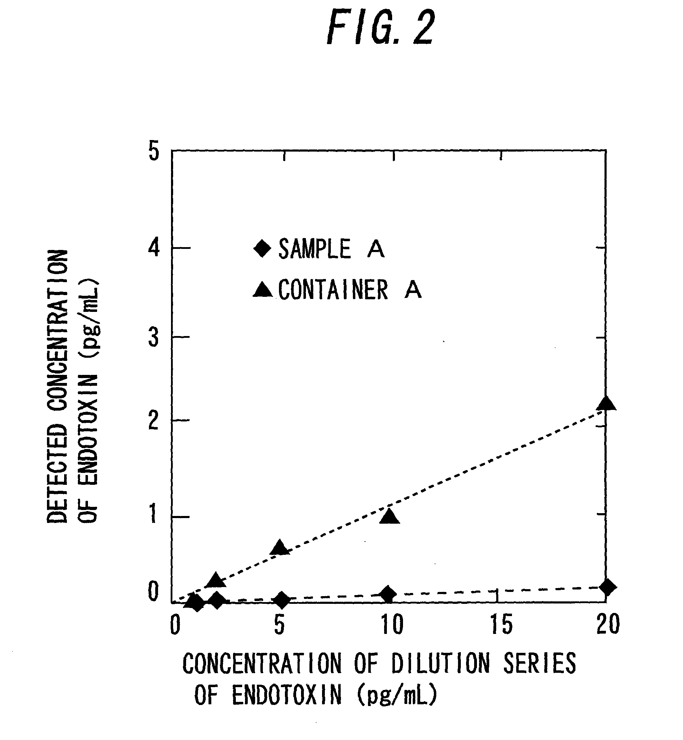

[FIG. 2] A graph illustrating relationships between the concentration of endotoxin in a sample, and the concentration of endotoxin determined in a glass container and the concentration of endotoxin determined in the sample not adsorbed and discharged from the glass container in the case where endotoxin is preliminarily adsorbed and immobilized to the glass container used for Examples of the present invention.

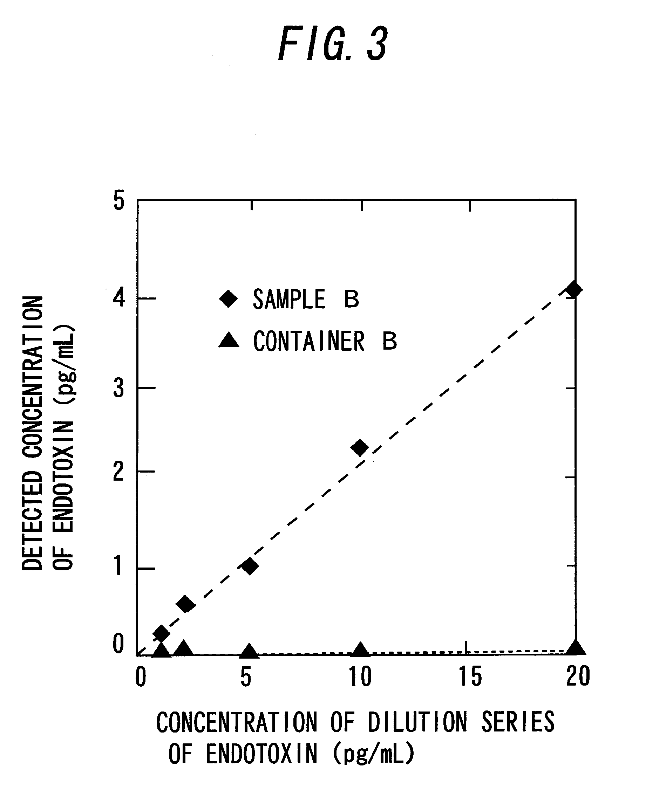

[FIG. 3] A graph illustrating relationships between the concentration of endotoxin in a sample, and the concentration of endotoxin determined in a glass container and the concentration of endotoxin determined in the sample not adsorbed and discharged from the glass container in the case where endotoxin is not preliminarily adsorbed and immobilized to the glass container used for Examples of the present invention.

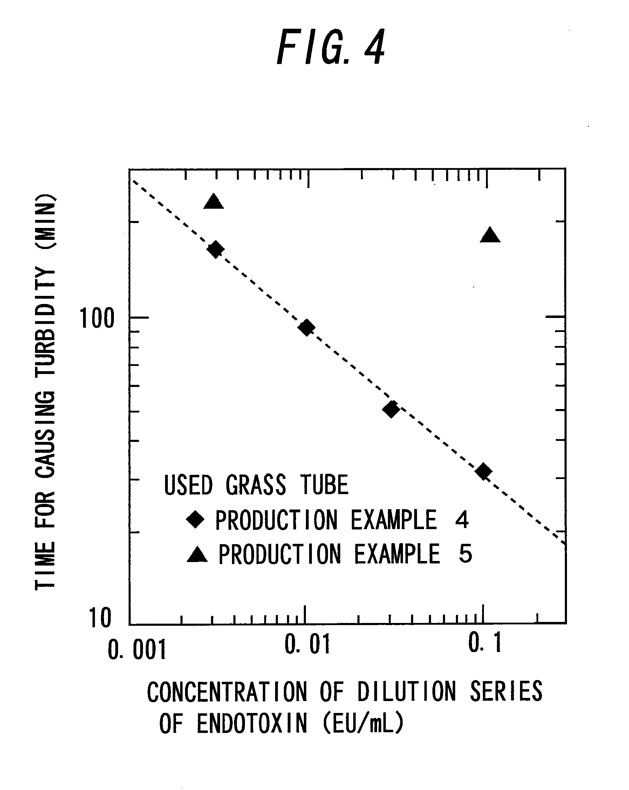

[FIG. 4] A graph illustrating a difference between the time to detect the turbidity in a reaction between endotoxin and LAL in the case where endotoxin is preliminarily adsorbed and immobilized to the glass tube used for Examples of the present invention and that in the case where endotoxin is not adsorbed and immobilized.

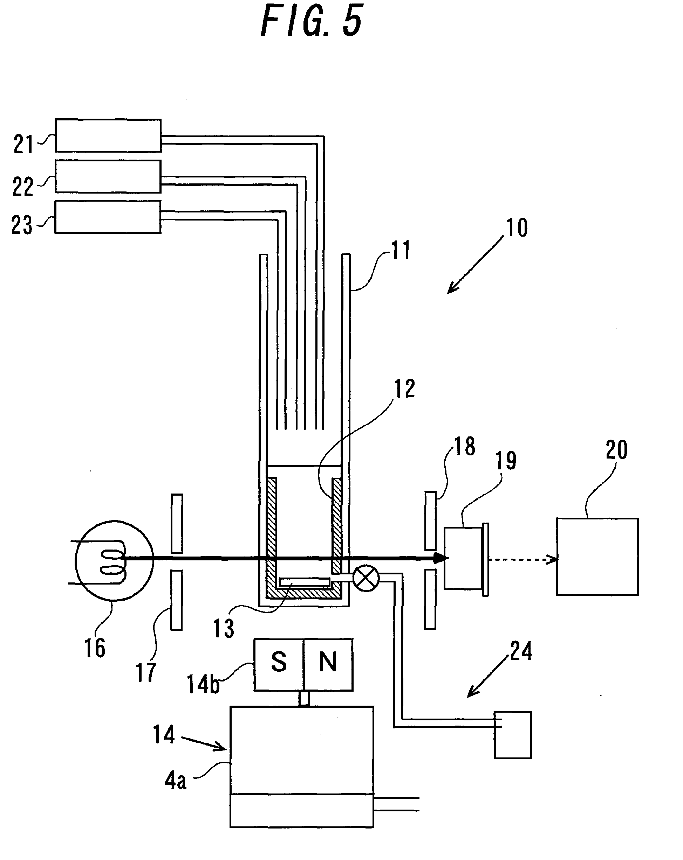

[FIG. 5] A conceptual diagram of a turbidimetric determination apparatus used for Examples of the present invention.

### Description of Embodiments

The process of forming a gel by a reaction between LAY and endotoxin has been studied well. That is, when endotoxin is bound to a serine protease, i.e., C-factor in LAL, the C-factor is activated, the activated C-factor hydrolyzes and activates another serine protease, i.e., B-factor in LAL to its activated form. The activated B-factor immediately hydrolyzes a precursor of a clotting enzyme in LAL to clotting enzyme as an activated form, and the clotting enzyme hydrolyzes a coagulogen in LAL to form a coagulin. Further, the formed coagulin is then associated with one another to form an insoluble gel.

In addition, when β-D-glucan is bound to G-factor in LAY, G-factor is activated and converted into activated G-factor. The activated G-factor hydrolyzes a precursor of clotting enzyme in LAL to produce clotting enzyme. As a result, as is the case with the reaction between endotoxin and LAL, coagulin is generated, and the generated coagulin molecules are associated with each other to further generate an insoluble gel.

A series of reactions as described above are similar to the process of forming a fibrin gel via a serine protease such as Christmas factor or thrombin present in mammals. Such enzyme cascade reactions have a very strong amplification effect because even a very small amount of an activation factor activates the subsequent cascade in a chain reaction. Therefore, according to a method of determining endotoxin using LAL, it is possible to detect a very small amount (sub-pg/mL order) of endotoxin.

Examples of a determination method which can quantify endotoxin include the turbidimetric method and the laser light scattering particle counting method, as described above. In such determination methods, endotoxin can be determined with a high sensitivity by detecting association products of a coagulin formed by the enzyme cascade reactions in LAL as the turbidity of a sample in the former method or as gel fine particles formed in the system in the latter method.

In particular, in the laser light scattering particle counting method, gel fine particles formed in the system are directly determined, and hence the method is more sensitive than the turbidimetric method. In addition, gel formation can be detected in a short time compared with the turbidimetric method because in general, a sample containing LAL and an analyte is forcibly stirred.

In addition, another method of determining a predetermined physiologically active substance further includes a colorimetric method. The method does not measure the turbidity of a sample caused by a coagulin gel although the method is based on the enzyme cascade reactions in LAL. The method is performed by determining absorbance changes caused by a reaction between an analyte and LAL containing a synthetic substrate, which is hydrolyzed by a clotting enzyme to develop a color. In the colorimetric method, the concentration of a chromogenic substance formed in the system is determined, and hence a low concentration of the predetermined physiologically active substance can be determined in a short time compared with the turbidimetric method or laser light scattering particle counting method, in both of which gel formation in a sample is determined.

However, in all the above-mentionedmethods, in the case where a substance which inhibits or promotes the reaction of LAIR (disrupter) is present, determination results are greatly affected.

In the present invention, a reaction between a concentrated predetermined physiologically active substance and LAL is performed after concentration of the predetermined physiologically active substance by adsorbing only the predetermined physiologically active substance in a sample containing the predetermined physiologically active substance to the observation region to be determined and removal of substances which affect the reaction of LAL. The procedure enables easy and accurate determination of the predetermined physiologically active substance in the sample without cumbersome pretreatments of the sample. Moreover, the amount of LAL required for determination can be reduced to about one-tenth at maximum by performing the reaction between the concentrated predetermined physiologically active substance and LAL not in a large reaction container but in a limited small region made of a resin or glass.

Hereinafter, best modes for carrying out this invention are described illustratively in detail with reference to drawings. However, the present invention is not limited to the following modes. In addition, the following description is made mainly for endotoxin as the predetermined physiologically active substance, but the same concept can be applied to β-D-glucan.

### Examples

First, the outline of determination of endotoxin in this example is described with reference to FIGS. 1. The material of the determination container 1 for concentrating endotoxin as shown in FIG. 1 (a) is desirably one which does not inhibit transmission of light with a wavelength to be used for determination because the turbidity or the like caused by a reaction between endotoxin concentrated by adsorption and LAL is optically determined. For example, the material is preferably a clear member made of glass, a polystyrene resin, an acrylic resin, a polyethylene resin, a polypropylene resin, or a PET resin.

Further, the cross-sectional shape of the determination container 1 is desirably rectangle, square, circle, ellipse, etc. Meanwhile, if the inner diameter of the container is too small, the light transmission rate varies little, while if the inner diameter is too large, endotoxin bound to the inside of the wall may act insufficiently for aggregating LAL. According to the conditions, the inner diameter of the container is preferably within a range of 0.1 mm to 10 mm, and more preferably within a range of 0.5 mm to 5 mm.

Next, in the case where the determination container 1 for determining endotoxin is a large scale container, the container may be designed so that a sample in the container can be stirred to uniformize the reaction between endotoxin and LAL. In this case, the shape of the determination container 1 is desirably one having an opened top and a closed bottom as shown in FIGS. 1. The adsorptive substance 2 to which endotoxin is adsorbed is desirably immobilized on a site corresponding to the observation region la in the determination container 1. Examples of the adsorptive substance 2 which adsorbs endotoxin include polymyxin B, polylysine, polyornithine, polyethyleneimine, a kind of silane coupling agent containing an amino group, histidine, and an anti-endotoxin antibody.

In this example, as shown in FIG. 1(a), a sample for determination of endotoxin is introduced into the determination container 1 in which the adsorptive substance 2 has been immobilized. Then, as shown in FIG. 1 (b), the endotoxin 4 in the sample is adsorbed to the adsorptive substance 2 and concentrated. In this example, this step corresponds to the adsorption step. Further, in this example 1, the determination container 1 corresponds to the reaction container.

Next, the sample is removed from the determination container 1, and the container is washed with washing water containing no endotoxin. As a result, as shown in FIG. 1(c), the determination container 1 in which the endotoxin 4 has been adsorbed to the adsorptive substance 2 and redundant sample is not contained is completed. In this example, this step corresponds to the removal step. Thereafter, as shown in FIG. 1 (d), an LAL reagent is introduced into the determination container 1 in such state, and a reaction between the adsorbed and concentrated endotoxin 4 and LAL is started. Further, on this occasion, the mixture of the endotoxin and LAL is stirred by moving the stainless steel magnetic stirring bar 3 by applying an electromagnetic force from the outside. In this example, this step corresponds to the reaction step.

Then, as the gel particles 5 are generated as shown in FIG. 1(e), the gel particles 5 are detected optically from the outside of the determination container 1 to detect endotoxin or to measure the concentration of endotoxin. In this example, this step corresponds to the detection step.

### <Production Example>

Hereinafter, production examples of instruments for determining endotoxin in this embodiment and a determination instrument for comparison are described. The following production examples are shown for illustrative purposes, and the instruments for determination according to the present invention are not limited to the following ones.

### (Production Example 1: production of stainless steel magnetic stirring bar from which no iron ion is eluted)

A stainless steel magnetic stirring bar (length: 4.5 mm, ϕ1 mm) was placed in a porcelanic crucible, and a heat treatment was performed in a muffle furnace at 650°C for 1 hour to form an oxide film with a thickness of 100 nm or more on the surface of the stirring bar, to thereby produce a stainless steel magnetic stirring bar fromwhichno iron ionwas eluted. Iron ion is known to act on endotoxin to inhibit the activity of endotoxin. Therefore, when the stainless steel magnetic stirring bar produced in this production example is used, deactivation of endotoxin due to iron ion can be avoided.

### (Production Example 2: production of glass container for immobilizing endotoxin)

0 0 µL of an aqueous solution of 0.01% poly-L-lysine was charged in a dry-heat-sterilized (250°C, 3 hours) glass cylindrical container with an inner diameter of 5 mm, an outer diameter of 7 mm, and a length of 50 mm, and the container was left to stand at room temperature for 2 hours to bind poly-L-lysine to the glass inner wall, which is an adsorptive substance. Thereafter, the aqueous solution of poly-L-lysine in the container was collected, and the inside of the container was washed with water for injection containing no endotoxin to remove an excessive amount of poly-L-lysine. The cylindrical container was dried at room temperature so that endotoxin is not brought into contact with the container, and one piece of the stainless steel magnetic stirring bar obtained in Production Example 1 was placed in the container. Moreover, the container was covered with a dry-heat-sterilized aluminum cap to seal the opening section on the top surface.

### (Production Example 3: production of glass container which does not adsorb endotoxin)

One piece of the stainless steel magnetic stirring bar obtained in Production Example 1 was placed in a glass cylindrical container with an inner diameter of 5 mm, an outer diameter of 7 mm, and a length of 50 mm (equivalent of the container used for Production Example 2), and the container was covered with the aluminum cap 4 to seal the opening section on the top surface, followed by dry-heat sterilization (250°C, 3 hours).

### (Production Example 4: production of glass tube which adsorbs endotoxin)

15 µL of an aqueous solution of 0 . 03% poly-L-lysine was charged in a dry-heat-sterilized (250°C, 3 hours) glass tube with an inner diameter of 1.0 mm, an outer diameter of 1.5 mm, and a length of 70 mm from an open end on one side, and the glass tube was fixed so that the injection end side was slightly lower than the other side to accumulate the liquid on the injection end side. Then, the solution was allowed to react at room temperature for 2 hours so that the solution was not brought into contact with endotoxin, to thereby bind poly-L-lysine only on the injection end side. Subsequently, an excessive amount of the aqueous solution of poly-L-lysine in the glass tube was collected, and the tube was dried at room temperature. Then, the glass tube was placed in a dry-heat-sterilized glass container (ϕ 10 mm, length 75 mm), and the opening portion of the glass container was sealed with a dry-heat-sterilized aluminum cap.

### (Production Example 5: production of glass tube which does not adsorb endotoxin)

A glass tube with an inner diameter of 1. 0 mm, an outer diameter of 1.5 mm, and a length of 70 mm was placed in a glass container (ϕ10 mm, length 75 mm), and the opening portion of the glass container was sealed with a dry-heat-sterilized aluminum cap. The glass container was dry-heat-sterilized (250°C, 3 hours).

### <Determination Example>

Hereinafter, determination examples are described for comparing the instruments for determining endotoxin described in the above-mentioned production examples with a determination instrument for comparison with regard to the performance as instruments for determining endotoxin.

### (Determination Example 1)

Endotoxin dilution series were prepared by diluting a standard endotoxin with water for injection. 200 µL of the dilution series were charged in the glass container obtained in Production Example 2, and the solution was stirred at 37°C for 20 minutes using the stainless steel magnetic stirring bar in the container to adsorb endotoxin in a sample to poly-L-lysine on the inner wall of the glass container.

Next, the aqueous solution of endotoxin in the glass container was collected (the collected sample was defined as Sample A), and the inside of the container was lightly washed with 300 µL of water for injection. Washing was carried out twice in total (the thus-prepared container was defined as Container A). Subsequently, Sample A (200 µL) was mixed with an LAL reagent (manufactured by Wako Pure Chemical Industries, Ltd., Limulus ES-II Single Test Wako), and the mixture was charged in the glass container obtained in Production Example 3, followed by determining a process of aggregation of the LAL reagent by the laser light scattering particle counting method (the reaction was carried out at 37°C).

Moreover, the LAL reagent was dissolved in water for injection (200 µL), and the solution was charged in Container A, followed by determining the process of aggregation of the LAL reagent by the laser light scattering particle counting method in the same way as described above. FIG. 2 shows the results. As shown in FIG. 2, the concentration of endotoxin detected by the reaction with the LAL reagent in Container A increased linearly in accordance with the increased concentration of endotoxin adsorbed and immobilized on Container A. On the other hand, the concentration of endotoxin not adsorbed and immobilized on Container A, i.e., the concentration of endotoxin in Sample A was lower overall compared with the concentration of endotoxin in Container A.

### (Determination Example 2)

200 µL of the endotoxin dilution series prepared in the same way as in Determination Example 1 were charged in the glass container obtained in Production Example 3 and stirred at 37°C for 20 minutes using the stainless steel magnetic stirring bar included in the container and prepared in Production Example 1. Thereafter, the aqueous solution of endotoxin in the glass container was collected (the collected sample was defined as Sample B), and the inside of the container was lightly washed with 300 µL of water for injection. Washing was carried out twice in total (the thus-prepared container was defined as Container B).

Subsequently, Sample B (200 µL) was mixed with the LAL reagent, and the mixture was charged in another glass container obtained in Production Example 3, followed by determining the process of aggregation of the LAL reagent by the laser light scattering particle counting method (the reaction was carried out at 37°C). Moreover, the LAL reagent was dissolved in water for injection (200 µL), and the solution was charged in Container B, followed by determining the process of aggregation of the LAL reagent by the laser light scattering particle counting method in the same way as described above. FIG. 3 shows the results. As shown in FIG. 3, endotoxin was not detected even by using the LAL reagent reacted in Container B because endotoxin was not adsorbed and immobilized on Container B. On the other hand, the concentration of endotoxin in Sample B, i.e., the concentration of endotoxin which was not adsorbed was high, and further, increased linearly in proportion to the concentration of endotoxin treated because endotoxin was not adsorbed and immobilized on Container B.

### (Determination Example 3)

The glass tube obtained in Production Example 4 was inserted into a Teflon (registered trademark) resin joint (in which two opening sections were provided linearly, one was a circular opening section (ϕ 1.5 mm) into which the glass tubes used for Production Examples 4 and 5 were able to be inserted, and the other was a circular opening section (ϕ 4.0 mm) into which an outlet port of a disposable injector were able to be inserted) so that the open end side treated with poly-L-lysine was located on the joint side.

A disposable tuberculin syringe (TERUMO CORPORATION, volume 1.0 mL) was connected to the other hole of the joint. The unconnected end of the glass tube was placed in an endotoxin dilution series solution (1 mL) prepared in the same way as in Determination Example 1, and the syringe was reciprocated (stroke length=40 mm, number of strokes=50 times, stroke rate=4 times/min) so that endotoxin in the sample passed through the inside of the glass tube and was brought into contact with the part treated with poly-L-lysine. In a glass tube untreated with poly-L-lysine obtained in Production Example 5, the sample was treated in the same way as described above.

Next, 400 µL of water for injection was caused to pass through the respective glass tubes to remove unbound endotoxin, and 20 µL of the LAL reagent dissolved in water for injection was charged from the opening end side treated with poly-L-lysine. The opening end side was inserted into putty for a hematocrit tube by 1.5 mm to seal the tubes. Changes in light transmission rates at sites located at 6 mm from the sealed end side of the glass were determined (the reaction was carried out at 37°C). In the glass tube obtained in Production Example 5, the treatment was carried out, followed by determination in the same way as described above.
FIG. 4 shows the results.

As shown in FIG. 4, in the glass tube obtained in Production Example 4 (adsorbing endotoxin), the turbidity of the sample was detected in a relatively short time, and the time to detect the turbidity was reduced as the concentration of endotoxin treated increased. On the other hand, in the glass tube obtained in Production Example 5 (adsorbing no endotoxin), it was observed that the time to detect the turbidity was reduced as the concentration of endotoxin treated increased. However, the time to detect the turbidity became longer overall.

As described above, it is found that determination of the concentration of endotoxin in the glass tube obtained in Production Example 4 (adsorbing endotoxin) can be carried out in a short time compared with the glass tube obtained in Production Example 5 (adsorbing no endotoxin).

Here, in the above-mentioned examples, as shown in FIG. 1 (a), a sample for determination of endotoxin was introduced manually into the determination container 1 on which the adsorptive substance 2 was immobilized. Moreover, the sample was removed manually from the determination container 1, and the container was washed with washing water containing no endotoxin. In addition, as shown in FIGS. 1 (c) and 1 (d), the LAL reagent was introduced manually into the determination container 1 in which endotoxin 4 was adsorbed to the adsorptive substance 2 and an excessive amount of the sample was not present.

However, the above-mentioned steps may be carried out automatically or semi-automatically using a special apparatus. In such case, it is possible to form an apparatus for completing determination of endotoxin in a sample only by setting a reaction container having an adsorption part to which an adsorptive substance is bound by a measurer.

FIG. 5 illustrates a conceptual diagram of a turbidimetric determination apparatus 10 as an apparatus for determining endotoxin in this example. In the turbidimetric determination apparatus 10 in this example, endotoxin is determined by the stirring turbidimetric method. In this example, the adsorptive substance 12 capable of adsorbing endotoxin is immobilized on the determination container 11 made of clear glass. A stainless steel stirring bar 13 on which an oxide film with a thickness of 100 nm or more is formed is provided in the determination container 11. A stirrer 14 is placed on the lower side of the determination container 11. In the stirrer 14, a permanent magnet 14b is rotated by applying a current to a motor 14a, and a stainless steel stirring bar 13 is rotated by the action of rotating magnetic field.

It should be noted that the turbidimetric determination apparatus 10 is equipped with a light source 16 and a light receiving element 19. Light which exits from the light source 16 passes through an aperture 17, and then enters a sample in the determination container 11. The light which has transmitted through the sample in the determination container 11 passes through an aperture 18, and then the light receiving element 19 is irradiated with the light. The light receiving element 19 outputs a photoelectric signal based on the intensity of the received light. The output of the photoelectric signal is input into an arithmetic apparatus 20. In the arithmetic apparatus 20, judgment of the reaction starting time and determination of the concentration of endotoxin are carried out according to a program (algorithm) preliminarily stored.

In the turbidimetric determination apparatus 10, before starting of the determination, a sample for determination of endotoxin is introduced into the determination container 11 by an endotoxin introduction apparatus 21. On this occasion, endotoxin is adsorbed to the adsorptive substance 12 and concentrated. Next, the sample containing endotoxin not adsorbed to the adsorptive substance 12 is discharged from the determination apparatus 11 by a discharge apparatus 24.

Then, washing water containing no endotoxin is introduced into the determination apparatus 11 from a washing apparatus 22 and is discharged by the discharge apparatus 24, and the procedure is repeated several times to wash the inside of the determination container 11. Thereafter, an adsorption reagent containing LAL is introduced into the determination container 11 by an LAL reagent introduction apparatus 23. As a result, a reaction between endotoxin adsorbed to the adsorptive substance 12 and LAL is started.

Thereafter, rotation of the stirring bar 13 and emission of the light source 16 are started to start determination of the concentration of endotoxin by the stirring turbidimetric method.

In the above-mentioned apparatus, the determination container 11 corresponds to the reaction container. The endotoxin introduction apparatus 21 corresponds to the adsorption means. The washing apparatus 22 and discharge apparatus 24 correspond to the removal means. The LAL reagent introduction apparatus 23 corresponds to the LAL introduction means. In addition, the detection means includes the light source 16, apertures 17 and 18, light receiving element 19, arithmetic apparatus 20, stirring bar 13, and stirrer 14. It should be noted that the turbidimetric determination apparatus 10 is shown as an example of the determination apparatus for a physiologically active substance of biological origin, and stirring of a sample using the stirring bar 13 and stirrer 14 is not always necessary. Of course, a method of detecting gel particles generated by the reaction between endotoxin and LAL by scattering light may be employed as a method of detecting endotoxin. The configurations of the adsorption means, removal means, and LAL introduction means are not limited to the above-mentioned configurations.

It should be noted that, in Examples above, as endotoxin was determined, polymyxin B, polylysine, polyornithine, polyethyleneimine, a kind of silane coupling agent containing an amino group, histidine, and an anti-endotoxin antibody were given as examples of the adsorptive substance 2. However, in addition to those, an inorganic substance such as calcium carbonate, aluminum oxide, or hydroxyapatite maybe used. When such inorganic substance is used as the adsorptive substance 2, good adsorbability can be achieved regardless of the physiologically active substance to be determined.

Further, in particular, in the case where β-D-glucan is determined, an alkyl group-containing hydrophobic substance, a derivative of the alkyl group-containing hydrophobic substance, aniline blue, cellulose, a derivative of cellulose, glucan, or a derivative of glucan may be used as the adsorptive substance 2. The alkyl group (such as octadecyl group)-containing hydrophobic substance and the derivative thereof are known to preferably adsorb glucan. Meanwhile, aniline blue is known to have a structure where β-1,3- and β-1,4- bond moieties in the sugar chain form a complex. Moreover, cellulose and the derivative thereof and glucan and the derivative thereof are considered to easily adsorb β-D-glucan via a hydrogen bond or the like because the affinity of polysaccharides is high. In addition, an antibody which specifically adsorbs β-D-glucan may be used as the adsorptive substance 2.

Further, in the case where endotoxin is determined like Examples above, the effect of β-D-glucan on determination of endotoxin can be removed by using an existing LAL reagent prepared so as to avoid determination of β-D-glucan. On the other hand, in the case where β-D-glucan is determined, it is necessary to take measures for removing the effect of endotoxin on determination. Specific examples of the measures include: use of an LAL reagent from which C-factor reacting with endotoxin has been removed; use of a sample, from which endotoxin has been removed with polymyxin B or another amino group-containing polymer compound which can remove endotoxin at a high level, in determination; and addition of an endotoxin binding site for C-factor (sushi region) to the LAL reagent.

### Reference Signs List

- 1: determination container
- la: observation region
- 2: adsorptive substance
- 3: stainless steel magnetic stirring bar
- 4: endotoxin
- 5: gel particle
- 10: turbidimetric determination apparatus
- 11: determination container
- 12: adsorptive substance
- 13: stirring bar
- 14: stirrer
- 16: light source
- 17, 18: aperture
- 19: light receiving element
- 20: arithmetic apparatus
- 21: endotoxin introduction apparatus
- 22: washing apparatus
- 23: LAL reagent introduction apparatus
- 24: discharge apparatus

## Claims

1. A method of determining a physiologically active substance of biological origin, which is used for detecting the physiologically active substance of biological origin in a sample or determining a concentration of the physiologically active substance of biological origin in the sample by reacting the physiologically active substance of biological origin in the sample with LAL which is a limulus amoebocyte lysate, **characterized in that**:
an adsorption step of adsorbing the physiologically active substance of biological origin in the sample to a predetermined adsorptive substance capable of adsorbing the physiologically active substance of biological origin;
a removal step of removing the sample excluding the physiologically active substance of biological origin adsorbed to the adsorptive substance in the adsorption step from the adsorptive substance, which is carried out after the adsorption step;
a reaction step of reacting the physiologically active substance of biological origin adsorbed to the adsorptive substance in the adsorption step with LAL, which is carried out after the removal step; and
a detection step of optically detecting gelation or formation of gel particles of LAL in the reaction step.

2. A method of determining a physiologically active substance of biological origin according to claim 1, wherein the physiologically active substance of biological origin is endotoxin or β-D-glucan.

3. A method of determining a physiologically active substance of biological origin according to claim 1, wherein the predetermined adsorptive substance comprises at least one of calcium carbonate, aluminum oxide, and hydroxyapatite.

4. A method of determining a physiologically active substance of biological origin according to claim 1, wherein the physiologically active substance of biological origin comprises endotoxin, and the predetermined adsorptive substance comprises at least one of polymyxin B, polylysine, polyornithine, polyethyleneimine, a kind of silane coupling agent containing an amino group, histidine, and an anti-endotoxin antibody.

5. A method of determining a physiologically active substance of biological origin according to claim 1, wherein the physiologically active substance of biological origin comprises β-D-glucan, and the predetermined adsorptive substance comprises at least one of an alkyl group-containing hydrophobic substance, a derivative of the alkyl group-containing hydrophobic substance, aniline blue, cellulose, a derivative of cellulose, glucan, and a derivative of glucan.

6. A kit for determining a physiologically active substance of biological origin, which is used for optical detection of gelation or formation of gel particles caused by a reaction between the physiologically active substance of biological origin in a sample and LAL which is a limulus amoebocyte lysate, **characterized in that**:
a predetermined adsorptive substance capable of adsorbing the physiologically active substance of biological origin is bound to an inside of a container or tubular member formed of a material capable of transmitting light to be used for the optical detection.

7. A kit for determining a physiologically active substance of biological origin according to claim 6, wherein the inside of the container is equipped with a stainless steel magnetic stirring bar on which an oxide film with a thickness of 100 nm or more is formed.

8. A kit for determining a physiologically active substance of biological origin according to claim 6 or 7, wherein the predetermined adsorptive substance comprises at least one of calcium carbonate, aluminum oxide, and hydroxyapatite.

9. A kit for determining a physiologically active substance of biological origin according to claim 6 or 7, wherein the physiologically active substance of biological origin comprises endotoxin, and the predetermined adsorptive substance comprises at least one of polymyxin B, polylysine, polyornithine, polyethyleneimine, a kind of silane coupling agent containing an amino group, histidine, and an anti-endotoxin antibody.

10. A kit for determining a physiologically active substance of biological origin according to claim 6 or 7, wherein the physiologically active substance of biological origin comprises β-D-glucan, and the predetermined adsorptive substance comprises at least one of an alkyl group-containing hydrophobic substance, a derivative of the alkyl group-containing hydrophobic substance, aniline blue, cellulose, a derivative of cellulose, glucan, and a derivative of glucan.

11. An apparatus for determining a physiologically active substance of biological origin, which is used for detecting the physiologically active substance of biological origin in a sample or determining a concentration of the physiologically active substance of biological origin in the sample by optical detection of gelation or formation of gel particles caused by a reaction between the physiologically active substance of biological origin in the sample and LAL which is a limulus amoebocyte lysate, **characterized in that**:
a reaction container, which is formed of a material capable of transmitting light to be used for the optical detection and has an adsorption part for adsorbing the physiologically active substance of biological origin in the sample in an inside of the container;
adsorption means for adsorbing the physiologically active substance of biological origin in the sample to the adsorption part by introducing the sample into the reaction container;
removal means for removing the sample excluding the physiologically active substance of biological origin adsorbed to the adsorption part from the reaction container;
LAL introduction means for introducing LAL into the reaction container in which the physiologically active substance of biological origin in the sample is adsorbed to the adsorption part;
and
detection means for optically detecting gelation or formation of gel particles of LAL introduced by the LAL introduction means.

12. An apparatus for determining a physiologically active substance of biological origin according to claim 11, comprising:
a stainless steel magnetic stirring bar, on which an oxide film with a thickness of 100 nm or more is formed, in an inside of the reaction container; and
stirring means for stirring the sample by rotating the magnetic stirring bar through application of an electromagnetic force to the magnetic stirring bar from an outside of the reaction container.

13. An apparatus for determining a physiologically active substance of biological origin according to claim 11 or 12, wherein the absorption part is formed by binding at least one of calcium carbonate, aluminum oxide, and hydroxyapatite to the reaction container.

14. An apparatus for determining a physiologically active substance of biological origin according to claim 11 or 12, wherein the physiologically active substance of biological origin comprises endotoxin, and the absorption part is formed by binding at least one of polymyxin B, polylysine, polyornithine, polyethyleneimine, a kind of silane coupling agent containing an amino group, histidine, and an anti-endotoxin antibody to the reaction container.

15. An apparatus for determining a physiologically active substance of biological origin according to claim 11 or 12, wherein the physiologically active substance of biological origin comprises β-D-glucan, and the absorption part is formed by binding at least one of an alkyl group-containing hydrophobic substance, a derivative of the alkyl group-containing hydrophobic substance, aniline blue, cellulose, a derivative of cellulose, glucan, and a derivative of glucan to the reaction container.
